# EUROPEAN PATENT APPLICATION

(11) **EP 3 878 943 A1**
(43) Date of publication of application: **15.09.2021**
(21) Application number: 20162810.4
(22) Date of filing: 12.03.2020
(51) Int. Cl.: C12N 1/14, A01G 18/00, E04B 1/14, E04B 1/16, E04B 1/74

(54) **METHOD OF MANUFACTURING A PREFAB CONSTRUCTION ELEMENT**

(71) Applicant: FS-Insulation B.V., 3011 TA Rotterdam (NL)
(72) Inventor: Borra, Hans, 3707 TB Zeist (NL); van Driel, Roland, 7827 TB Emmen (NL)
(74) Representative: De Vries & Metman

(57) **Abstract**

The invention relates to a method of manufacturing a prefab construction element (11), preferably a load-bearing element, for frame construction, such as wood frame construction, comprising the steps of providing a roofing, flooring or wall panel (2), which panel (2) comprises an enclosure (3), providing a fungus and a substrate, introducing or preparing a mixture (10) of the fungus and the substrate, in the enclosure (3) and allowing the fungus to grow to form a network of hyphae through the mixture (10) and into the walls (4-7) of the enclosure (3) to form a mycelium composite, and drying the composite while it remains in the enclosure (3) of the panel (2).

## Description

The invention relates to a method of manufacturing a prefab, i.e. prefabricated, construction element, preferably a load-bearing element, for frame construction, also known as framing, such as wood frame construction. The invention further relates to a prefab construction element.

WO 2008/073489 relates to a composite material that is comprised of a substrate of discrete particles and a network of interconnected mycelia cells bonding the discrete particles together. The composite material is made by inoculating a substrate of discrete particles and a nutrient material with a preselected fungus. The fungus digests the nutrient material over a period of time sufficient to grow hyphae and to allow the hyphae to form a network of interconnected mycelia cells through and around the discrete particles thereby bonding the discrete particles together to form a self-supporting composite material.

The method may be carried out in a batchwise manner by placing the mixture and inoculum in a form so that the finished composite material takes on the shape of the form. Alternatively, the method may be performed in a continuous manner to form an endless length of composite material.

In the example shown in Figure 7 of WO 2008/073489, stiff exterior faces are added to a rectangular panel, thus providing a "panelized system composed of a mycelia bonded core and exterior facing system can be created. This panelized system has superior strength characteristics due to the addition of stiff exterior faces."

"In another embodiment, samples have also been produced where the exterior faces are placed *in vitro* during the incubator process. The growth of the filamentous fungi directly bonds the exterior faces to the mycelia bonded composite core producing a panelized system that can be used immediately after drying."

WO 2018/014004 relates to forming fungal materials and fungal objects from those fungal materials, the method comprising the steps of growing a first fungal tissue in contact with a nutritive vehicle; supplying a porous material in contact with said first fungal tissue; directing growth of said fungal tissue through said porous material such that a portion of said fungal tissue comprises a first fungal material having first fungal hyphae; optionally incorporating composite material; directing a change in the composition or growth pattern of at least some of said first fungal hyphae; separating at least a portion of the first fungal material from said nutritive vehicle; obtaining a second fungal material having second fungal hyphae; and forming a fungal object by encouraging fused growth between said first fungal material and said second fungal material and optionally incorporating composite material.

It is an object of the present invention to provide an improved method of manufacturing a prefabricated construction element.

To this end, the method according to the present invention comprises the steps of
providing a roofing, flooring or wall panel, which panel comprises an enclosure,
providing a fungus and a substrate,
introducing or preparing a mixture of the fungus and the substrate - and optionally a nutrient, e.g. if more carbon is required in the substrate - in the enclosure and
allowing the fungus to grow, e.g. for a period in a range from 50 to 120 hours, preferably in a range from 70 to 110 hours, to form a network of hyphae through the mixture and into the walls of the enclosure to form a mycelium composite, and
drying the composite while it remains in the enclosure of the panel.

Thus, the roofing, flooring, or wall panel serves as a formwork or mold for the mycelium composite and remains (a major) part of the actual prefab panel. By manufacturing a prefab construction element in this way, the composite adapts in shape and bonds to the panel and contributes to the strength and stiffness of the prefab construction element to such an extent that the amount of material in the panel itself can be reduced. Also, because the panel and the mycelium together form a finished of semi-finished product, the mycelium need not be removed from its mold.

In an embodiment, the mixture is introduced in a layer having a thickness of at least 15 centimeters, preferably at least 20 centimeters.

Many roofing, flooring, or wall panels have a thickness of at least 20 cm, often 25 centimeters or more, and are provided with rock wool or glass wool to provide insulation. By using a relatively thick layer of the mixture, the panel exhibits sufficient heat and noise insulation, without requiring a further material, such as rock wool or glass wool.

In an embodiment, the panel is made of wood, fiberboard, plywood, or other cellulose based material.

To facilitate and optionally mechanize, e.g. robotize, manufacture, in an embodiment, the mixture is introduced into the enclosure in the form of bulk, e.g. from a hopper, or in the form of blocks, preferably blocks having a width and height that corresponds to the width and height of the enclosure or at least of the part of the enclosure in which they are introduced.

Suitable substrates for creating mycelium composite include wood materials, e.g. particles, such as saw dust and wood shavings, and materials from grain, maize, rice, or hemp.

Materials that can be added, e.g. up to a total amount of 40 wt% of the substrate in total, to the substrate include vegetable materials such as cucumber, peppers, grass, reed, beer broth, potato steam peel, root pulp and used growing substrates from greenhouses. Other examples are polystyrene, plastics, and cardboard materials, as well as inorganic materials such as perlite and vermiculite, preferably to obtain a substrate with a low carbon footprint to replace materials with a high carbon footprint.

Another embodiment, comprises the inclusion of reinforcements, such as reinforcing fibers, e.g. filaments of staple fibers, or rods or beams in the mixture.

The fungus digests the nutrient components in the substrate over a period of time sufficient to grow hyphae and to allow the hyphae to form a network of interconnected mycelia cells through and around the discrete particles in the substrate thereby bonding the discrete particles together to form a mycelium composite and bonding the composite to the walls of the enclosure.

The at least one fungus is preferably a white rot fungus and preferably one that grows relatively quickly and/or is able to accept materials that are strange to its habitat.

In an embodiment, the fungus or at least one of the fungi is selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

Suitable nutrients include sugar, oatflakes, flour, rejected food, and human and animal hair.

In an embodiment, during at least part of the growing of the hyphae, the enclosure is covered, in particular to prevent moisture from escaping, and/or the temperature of the mixture is maintained in a range from 15 to 24 degrees Celsius.

In a further aspect, the growth of the fungus is stopped, in particular inactivated or killed, by means of heating, reduced pressure (vacuum), freezing, radiation and/or drying.

The invention also relates to a prefab construction element, preferably a load-bearing element, for frame construction, such as wood frame construction, comprising a roofing, flooring or wall panel, which panel comprises an enclosure, which enclosure contains a mixture of at least one fungus and a substrate, wherein a network of hyphae has formed through the mixture and into the walls of the enclosure to form a mycelium composite. In an embodiment, the mycelium permanently binds the mycelium composite to the panels.

In an embodiment, the construction element comprises a layer of the mixture having a thickness of at least 15 centimeters, preferably at least 20 centimeters and/or the panel is made from wood, fiberboard, plywood, or other cellulose based material.

In another embodiment, the panel comprises rafters dividing the enclosure in sections, and roof slabs and/or vertical battens fixed, e.g. nailed, to the panel and optionally horizontal battens fixed to the vertical battens.

In another embodiment, the ratio of the weight of the panel and the weight of the mixture, i.e. the weight of the panel divided by the weight of the mixture is smaller than 0,6, preferably smaller than 0,5, preferably smaller than 0,45 and/or the rafters have a width less than 25 mm, preferably less than 20 mm.

The invention will now be explained in more detail with reference to the figures, which schematically show an embodiment according to the present invention.
Figure 1 is an isometric view of a traditional roofing panel and a roofing panel according to the present invention, both having an enclosure.
Figures 2 and 3 show the panels of Figure 1 wherein respectively glass wool and a mixture of substrate and fungus has been introduced in the enclosure, in the form of bulk (Figure 2) and in the form of blocks (Figure 3).
Figure 4 is an isometric view of a prefab construction element according to the present invention.

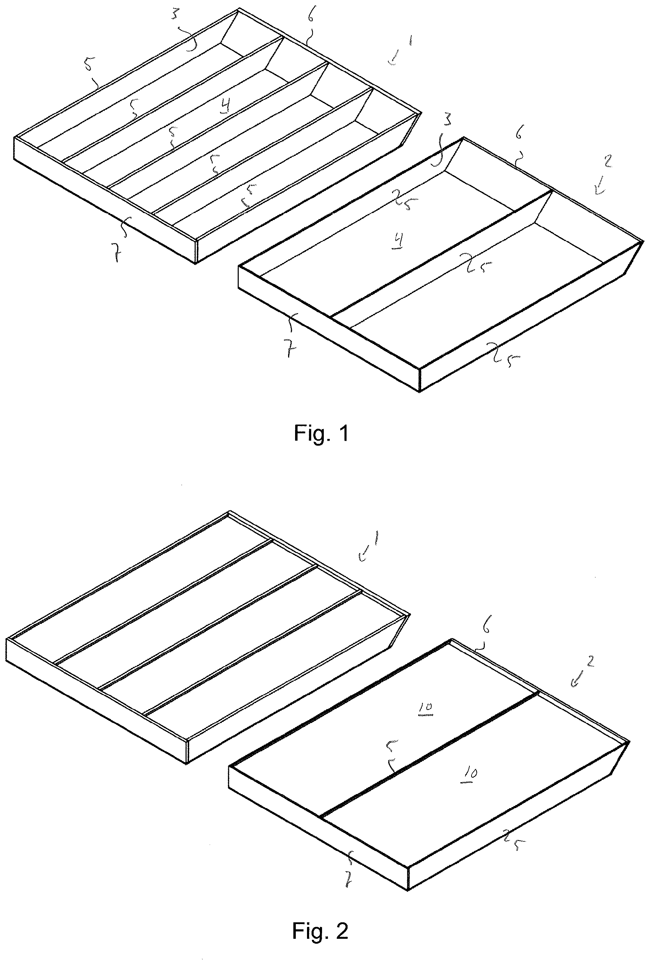

Figure 1 shows, on the left-hand side, a traditional roofing panel 1 and, on the right-hand side, a roofing panel 2 according to the present invention. Both panels 1, 2 have an enclosure 3 defined by a bottom wall 4, made e.g. of wood or fiberboard, side walls 5, and upper and lower end walls 6, 7, all made e.g. of wood. In this example, the upper end wall 6 forms a headboard that is at an inclination and that, once the panel is installed on a roof, forms the apex of the roof (inner) construction and supports e.g. a ridge beam and/or ridge tiles. The lower end wall 7 forms a gutter board.

The side walls 5 are in effect rafters that play a major role in providing strength and stiffness to the panel 1, 2. As shown in Figure 1, the traditional panel 1 comprises a total of five rafters 5, two on the sides (co)defining the enclosure 3 of the panel 1 and three inside the enclosure, dividing the enclosure into four sections. The panel 2 according to the present invention comprises a total of three rafters 5, two sides (co)defining the enclosure 3 of the panel 2 and one inside the enclosure dividing the enclosure into two sections.

After providing the panel, a substrate, such as a blend of hemp, foliage, and sawdust was mixed with a fungus (inoculum), for instance Pleurotus ostreatus, optionally at least one nutrient, such as oatflakes, and water.

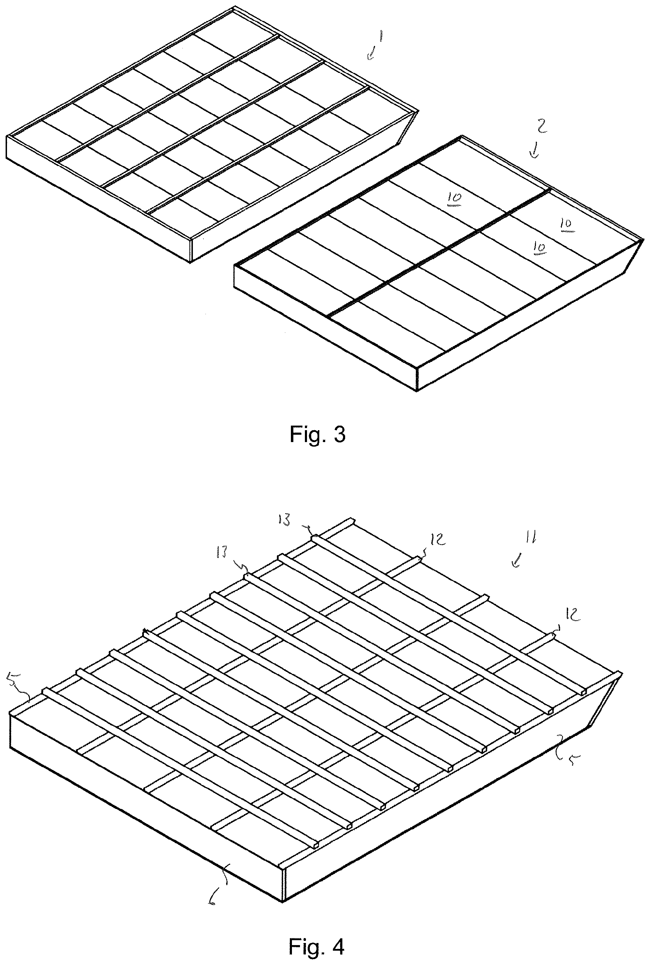

Figures 2 and 3 show the panel 2 according to the present invention with a mixture 10 of substrate and fungus having been introduced in the enclosure 3, in the form of bulk (Figure 2) and in the form of blocks (Figure 3).

After the mixture was introduced into the sections of the enclosure, the enclosure was covered, e.g. with an impermeable foil or tarpaulin, and the temperature of the mixture was maintained in a range from 15 to 24°C, for example 20°C. The fungus was allowed to grow, e.g. for a period in a range from 50 to 120 hours, preferably in a range from 70 to 110 hours, for example 100 hours, to form a network of hyphae through the mixture and into the walls 4-7 of the enclosure 3 to form a mycelium composite.

When the mycelium composite was considered at or near optimum, in terms of strength, stiffness and durability in a dried state, the fungus was killed by heating and drying the prefab construction element and the mycelium composite in it.

To extend the comparison of the construction element of the present invention with traditional construction elements, Figures 2 and 3 show, on the left-hand side, the traditional panel filled with glass wool as an insulating material. The panels 1, 2 have the same external dimensions and both fulfil official requirements (building codes) for strength, stiffness, and insulation. In a specific example, the traditional panel 1 has five rafters 5 having a height of 250 mm and a thickness of 32 mm, whereas the panel 2 according to the present invention has three rafters 5 having a height of 250 mm high and a thickness of 18 mm, saving more than 60% on the wood of the rafters alone.

Figure 4 shows a prefabricated construction element 11 wherein the panel 2 comprises, in addition to the rafters 5 forming the side walls and dividing the enclosure 3 in sections, vertical battens 12 fixed to the panel 2 and optionally horizontal battens 13 fixed to the vertical battens 14, thus ready to be installed in a wood frame building. Because the mycelium composite is liquid water repellent and water vapour permeable, the foil covering the glass wool in the traditional construction element can be omitted in the construction element according to the present invention.

The invention is not restricted to the above-described embodiments, which can be varied in a number of ways within the scope of the claims, and, for instance, applies similarly advantageously in construction elements for floors and walls.

## Claims

1. Method of manufacturing a prefab construction element (11), preferably a load-bearing element, for frame construction, such as wood frame construction, comprising the steps of
providing a roofing, flooring or wall panel (2), which panel (2) comprises an enclosure (3),
providing a fungus and a substrate,
introducing or preparing a mixture (10) of the fungus and the substrate, in the enclosure (3) and
allowing the fungus to grow to form a network of hyphae through the mixture (10) and into the walls (4-7) of the enclosure (3) to form a mycelium composite, and
drying the composite while it remains in the enclosure (3) of the panel (2).

2. Method according to claim 1, wherein the mixture (10) is introduced in a layer having a thickness of at least 15 centimeters, preferably at least 20 centimeters.

3. Method according to claim 1 or 2, wherein the panel (2) is made of wood, fiberboard, plywood, or other cellulose based material.

4. Method according to any one of the preceding claims, wherein the mixture (10) is introduced into the enclosure (3) in the form of bulk or in the form of blocks.

5. Method according to any one of the preceding claims, comprising the inclusion of reinforcements in the mixture.

6. Method according to any one of the preceding claims, wherein the fungus or at least one of the fungi is selected from the group consisting of Pleurotus ostreatus, Pleurotus eryngii, Stropharia Rugosoannulata, Trametes versicolor, Ganoderma Lucidum, Phanerochaete chrysosporium, Bjerkandera adusta, Lentinula edodes, Pycnoporus cinnabarinus, Pycnoporus sanguineus, Grifola frondosa, Schizophyllum commune, Neolentinus lepideus, and Heterobasidiom annosum.

7. Method according to any one of the preceding claims, wherein during at least part of the growing of the hyphae, the enclosure (3) is covered and/or the temperature of the mixture is maintained in a range from 15 to 24 degrees Celsius.

8. Method according to any one of the preceding claims, wherein the growth of the fungus is stopped by means of heating, reduced pressure, freezing, radiation and/or drying.

9. Method according to any one of the preceding claims, wherein a nutrient is added to the mixture.

10. Prefab construction element (11), preferably a load-bearing element, for frame construction, such as wood frame construction, comprising a roofing, flooring or wall panel (2), which panel (2) comprises an enclosure (3), which enclosure (3) contains a mixture (10) of at least one fungus and a substrate, wherein a network of hyphae has formed through the mixture (10) and into the walls (4-7) of the enclosure (3) to form a mycelium composite.

11. Prefab construction element (11) according to claim 10, comprising a layer of the mixture (10) having a thickness of at least 15 centimeters, preferably at least 20 centimeters.

12. Prefab construction element (11) according to claim 10 or 11, wherein the panel (2) is made of wood, fiberboard, plywood, or other cellulose based material.

13. Prefab construction element (11) according to any one of the claims 10-12, wherein the panel (2) comprises rafters (5) dividing the enclosure (3) in sections, and roof slabs and/or vertical battens (12) fixed to the panel and optionally horizontal battens (13) fixed to the vertical battens (12).

14. Prefab construction element (11) according to any one of the claims 13, wherein the rafters (5) have a thickness less than 25 mm, preferably less than 20 mm.

15. Prefab construction element (11) or method according to any one of the preceding claims, wherein the ratio of the weight of the panel (2) and the weight of the mixture (10) is smaller than 0,6, preferably smaller than 0,5, preferably smaller than 0,45.
